# EUROPEAN PATENT APPLICATION

(11) **EP 2 426 147 A1**
(43) Date of publication of application: **07.03.2012**
(21) Application number: 10758759.4
(22) Date of filing: 24.03.2010
(51) Int. Cl.: C07K 16/28, C12N 15/09, G01N 33/53

(54) **METHOD FOR PRODUCING ANTI-GPCR ANTIBODY AND ANTI-GPCR ANTIBODY**

(30) Priority: 31.03.2009 JP 2009083900
(71) Applicant: Mie University, Mie 514-8507 (JP)
(72) Inventor: TAMARU, Yutaka, Tsu-shi Mie 514-8507 (JP)
(74) Representative: Høiberg A/S
(86) International application number: PCT/JP2010/055783
(87) International publication number: WO 2010/113988

(57) **Abstract**

Disclosed is a discussion of a method for producing anti-mammalian GPCR antibody and of the antibody itself. Anti-mammalian GPCR antibody is produced through immunization involving exposure of fish to full-length or truncated mammalian GPCR, and anti-mammalian GPCR antibody that can be obtained by this method is also discussed.

## Description

### [Technical Field]

The present invention relates to a method of producing anti-G protein-coupled receptor (GPCR) antibody and the product, i.e., anti-GPCR antibody, of this method. In particular, the said GPCR is of mammalian origin.

### [Background Art]

GPCRs can bind drugs and are receptors that transduce signals initiated by the binding of biologically active substances, such as hormones, to the inside of the cell. GPCRs belong to a family of proteins within the signal transduction system that includes numerous protein types. Proteins of this family share a common feature, namely the presence of seven transmembrane domains within the plasma membrane (Non-Patent Document 1).
Many proteins of this family are orphan receptors, and thus, their agonists (ligands) have not been identified. However, recently, researchers have extensively studied these proteins as target molecules for therapeutic antibodies, diagnostic agents, and the like because they have demonstrated important functions in vivo.

These studies aimed to produce various anti-GPCR antibodies. However, GPCRs have a complex conformation as described above, and hence, the preparation of protein is difficult. Consequently, anti-GPCR antibodies may not be readily obtained by conventional methods of protein immunization.
Accordingly, a DNA vaccination method (DNA immunization method) has been developed in which an epitope (immunization region) of the target molecule is chosen in silico, an expression vector in which the cDNA of the epitope has been cloned is introduced into an animal, and a hybridoma is produced (Non-Patent Document 2). This method facilitates the production of various anti-GPCR antibodies, and various companies are selling the products. However, this method of producing antibodies has the disadvantage of multiple steps, such as selection of the epitope and production of the hybridoma, which are time consuming and labor intensive.

### [References]

### [Non-Patent Documents]

Non-Patent Document 1: Nature. 2007 Oct 25; 449(7165):1003-7. Epub 2007 Oct 14
Non-Patent Document 2: Seibutsu-kogaku Kaishi, 86(8), 384-386(2008).

### [Summary of Invention]

### PROBLEMS TO BE SOLVED BY THE INVENTION

The problems that will be solved by the present invention are the lack of a method of producing anti-mammalian GPCR antibody and the lack of anti-mammalian GPCR antibody that can be produced by this method.

### MEANS FOR SOLVING THE PROBLEMS

As a result of extensive research to identify a solution to the abovementioned problems, the inventor of the present invention found that anti-mammalian GPCR antibody can be produced by immunizing fish with full-length or truncated GPCR of mammalian origin, such as human GPCR. Immunization of fish in the present production method is very simple as it may employ the direct exposure of fish to *Escherichia coli* capable of expressing the full-length or truncated mammalian GPCR, which serves as the antigen.

The present invention relates to a method of producing anti-mammalian GPCR antibody, the anti-mammalian GPCR antibody produced by the production method, and the like, as described subsequently in items (1) to (12).
(1) A method of producing anti-mammalian GPCR antibody, comprising a step of immunizing fish with full-length or truncated mammalian GPCR.
(2) A method of producing anti-mammalian GPCR antibody according to the abovementioned item (1) that comprises a step of introducing an expression vector harboring a gene encoding the full-length or truncated mammalian GPCR into *E. coli* or yeast and a step of exposing the fish to the *E. coli* or yeast.
(3) A method of producing anti-mammalian GPCR antibody according to item (1) or (2) mentioned above, wherein the mammalian GPCR is human GPCR.
(4) A method of producing anti-mammalian GPCR antibody according to any one of items (1) to (3) mentioned above, wherein the mammalian GPCR is a human leucine-rich repeat-containing G protein-coupled receptor (LGR).
(5) A method of producing anti-mammalian GPCR antibody according to item (4) mentioned above, wherein the human LGR is human LGR3 or LGR4.
(6) A method of producing anti-mammalian GPCR antibody according to any one of items (1) to (5) mentioned above, wherein the fish is either zebrafish, goldfish, Carassius, or carp.
(7) Anti-mammalian GPCR antibody that can be obtained by the production method comprising a step of immunizing fish with full-length or truncated mammalian GPCR.
(8) Anti-mammalian GPCR antibody according to item (7) mentioned above, wherein the step of immunizing fish is a step of exposing the fish to *E. coli* or yeast in which an expression vector containing a gene encoding the full-length or truncated mammalian GPCR had been introduced.
(9) Anti-mammalian GPCR antibody according to item (7) or (8) mentioned above, wherein the mammalian GPCR is human LGR.
(10) Anti-mammalian GPCR antibody according to item (9) mentioned above, wherein the mammalian GPCR is human LGR3 or LGR4.
(11) Anti-human GPCR antibody according to any one of items (7) to (10) mentioned above, wherein the fish is either zebrafish, goldfish, Carassius, or carp.
(12) A method of detecting anti-human GPCR antibody using fish IgM antibody that is directly labeled with horseradish peroxidase (HRP).

### EFFECTS OF THE INVENTION

The present invention provides a method of producing anti-mammalian GPCR antibody and provides anti-mammalian GPCR antibody that can be obtained by this method. Anti-mammalian GPCR antibody obtained by the present invention may be a potential therapeutic antibody, diagnostic agent, and the like, whose target molecules are mammalian GPCRs, such as human GPCRs, to which the said antibody specifically binds.

### [Brief Description of Drawings]

[Fig. 1] Figure 1 shows the confirmation of the expression of human LGR3(LRR) and human LGR4(LRR). (EXAMPLES)
[Fig. 2] Figure 2 shows the confirmation of the expression of human LGR3(LRR) and human LGR4(LRR). (EXAMPLES)
[Fig. 3] Figure 3 shows the confirmation of the expression of human LGR3(LRR) and human LGR4(LRR). (EXAMPLES)
[Fig. 4] (A) Figure 4A shows the results of the assay of the expression of anti-human LGR3 antibody (EXAMPLES). (B) Figure 4B shows the result of fluorescence detection of anti-human LGR3 antibody (EXAMPLES).

### [Description of Embodiments]

The method of producing anti-mammalian GPCR antibody in the present invention includes any method that comprises a step of immunizing fish with full-length or truncated mammalian GPCR and that produces anti-GPCR antibody. The term "mammals" refers to any mammal, such as human, mouse, or rat.
The step of immunizing fish with full-length or truncated mammalian GPCR refers to either a process of immunization achieved by injecting a whole (i.e., full-length of the amino acid sequence) or part (i.e., polypeptide) of the mammalian GPCR protein into fish or to a process of introducing an expression vector harboring a gene encoding the full-length or truncated GPCR into *E. coli* or yeast and subsequently exposing the fish to the *E. coli* or yeast.
Truncated mammalian GPCR may be any part of GPCR that facilitates the production of anti-mammalian GPCR antibody, the preferred part being the entire or part of the leucine-rich repeat (LRR) portion located at the N-terminus of GPCRs.
Examples of such a part include the LRR portion of human LGR, a human GPCR, particularly the LRR portion of human LGR3 (Accession No. P16473 (SEQ ID No. 1), hereinafter referred to as human LGR3(LRR)), and the LRR portion of human LGR4 (Accession No. NM_018490.2 (SEQ ID No. 2), hereinafter referred to as human LGR4(LRR)).

Anti-mammalian GPCR antibody of the present invention encompasses any antibody that recognizes mammalian GPCR; it may be an antibody that recognizes a specific part of the mammalian GPCR. Moreover, any protein among GPCRs may be the target of the antibody. Examples of such a target are LGRs, and more specifically, LGR3, LGR4, and the like.

The fish used for production of anti-mammalian GPCR antibody of the present invention may be zebrafish, goldfish, Carassius, or carp.

A method of detecting anti-mammalian GPCR antibody of the present invention may be one that employs fish IgM antibody that is directly labeled with HRP and the like. Fluorescent substances may be any known substances, including 3,3'-diaminobenzidine tetrahydrochloride (Chemi-Lumi One, Nacalai, 07880-70) and the like. Anti-mammalian GPCR antibodies may be detected with high sensitivity using an antibody that is directly labeled with a fluorescent substance.

The present invention will be further described in detail by examples; however, these examples are not intended to limit the scope of the present invention.

### EXAMPLES

### 1. Preparation of antigen

### 1) Production of GPCR-expressing E. coli

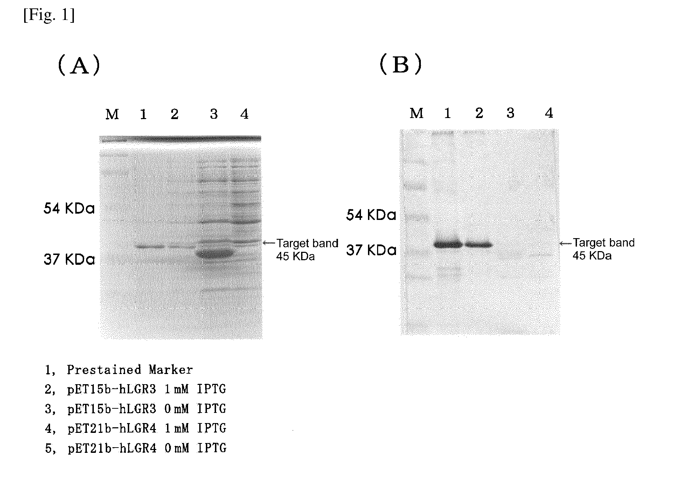

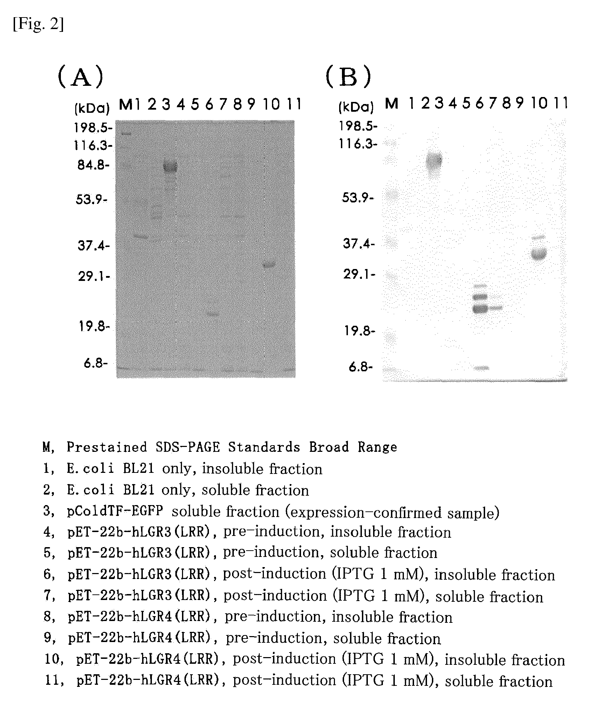

Human LGR3(LRR) or human LGR4(LRR), both of which are mammalian GPCRs, was incorporated into expression vectors (pET15b, pET21b, or pET22b; all from Novagen) leading to the construction of pET15b-hLGR3(LRR), pET22b-hLGR3(LRR), pET21b-hLGR4(LRR), and pET22b-hLGR4(LRR) as LGR3(LRR) or LGR4(LRR) expression vectors.
Each of the expression vectors prepared above was introduced into *E. coli* (BL21), and the expression of human LGR3(LRR) or human LGR4(LRR) was attempted. The presence (or absence) of human LGR3(LRR) or human LGR4(LRR) in proteins expressed from each bacteria was assayed by Western blotting using anti-His-tag antibody (GE Healthcare).
As shown in Figures 1 and 2, the expression of human LGR3(LRR) and human LGR4(LRR) was confirmed. The level of human LGR3(LRR) expression in *E. coli* incorporating the expression vector pET15b-hLGR3(LRR) was high with or without the addition of IPTG. In Figures 1 and 2, (A) shows the results of SDS-PAGE and (B) shows the results of Western blotting.
These results confirm that the *E. coli* to which the abovementioned expression vector is introduced may be used as an antigen.

### 2) Preparation of immunization feed

After culturing the antigen protein-expressing *E. coli* that was produced in step 1) above until O.D. reached approximately 0.5, expression of cells was induced (induction conditions: pET: 1 mM IPTG, 25°C, 4 hours; pColdTF: 0.1 mM IPTG, 15°C, 24 hours). The cells were then collected by centrifugation at 2500 ×*g* for 10 minutes.
Two milliliter of ampicillin (sodium salt, 100 mg/mL), 4 g TetraMin (registered trademark) powder, and 1 g *E. coli* (wet weight) were mixed, and the resultant paste was extruded from a "TERUMO (registered trademark)" syringe for feed preparation. The tip of the syringe was heated using a gas burner to seal the hole, and a hole was then made with a G21 needle. This produced a thread-like feed containing the *E. coli.* This feed was chopped into granules approximately 2 mm size and used as immunization feed.

### 2. Preparation of immunized fish (zebrafish)

Zebrafish were kept at a water temperature of 27.5°C, exposed to light for 14 hours, and then kept in darkness for 10 hours. Each test group comprised 50 fishes, and a total of 300 zebrafish (6 groups) were used. Each of 6 water baths (60 cm length, 30 cm width, and 30 cm height; each contained 50 fish) was filled with 20 L of environmental water.

### 3. Immunization

The zebrafish were exposed to human LGR3(LRR) by feeding on the immunization feed prepared in step 1. mentioned above.
Ten days after the first exposure, the second exposure was performed (The day of the first exposure is denoted as day 0). Blood was collected on day 16.
Production of antibodies against human LGR3 or human LGR4 because of the exposure was confirmed by dot blotting.

### 4. Confirmation

### 1) Preparation of proteins for antibody titer determination

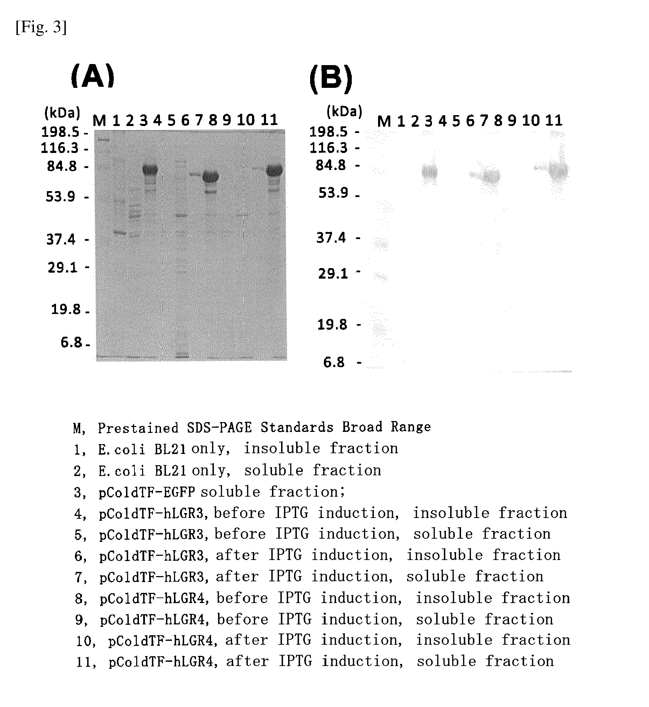

Human LGR3(LRR) or human LGR4(LRR) was inserted into an expression vector (pColdTF, TaKaRa), thus leading to the construction of pColdTF-hLGR3(LRR) or pColdTF-hLGR4(LRR) as human LGR3(LRR) or human LGR4(LRR) expression vectors.
Each of these vectors was introduced into *E. coli,* and human LGR3(LRR) or human LGR4(LRR) was expressed. The resultant proteins were purified and then analyzed by Western blotting using commercially available anti-His-tag antibody (GE Healthcare).
As shown in Figure 3, human LGR3(LRR) and human LGR4(LRR) were highly expressed in the soluble fraction. Figure 3(A) shows the results of SDS-PAGE and 3(B) shows the results of Western blotting. Of these, purified human LGR3(LRR) was used for confirming antibody expression as a protein standard (authentic preparation) of known concentration.

### 2) Confirmation of antibody expression

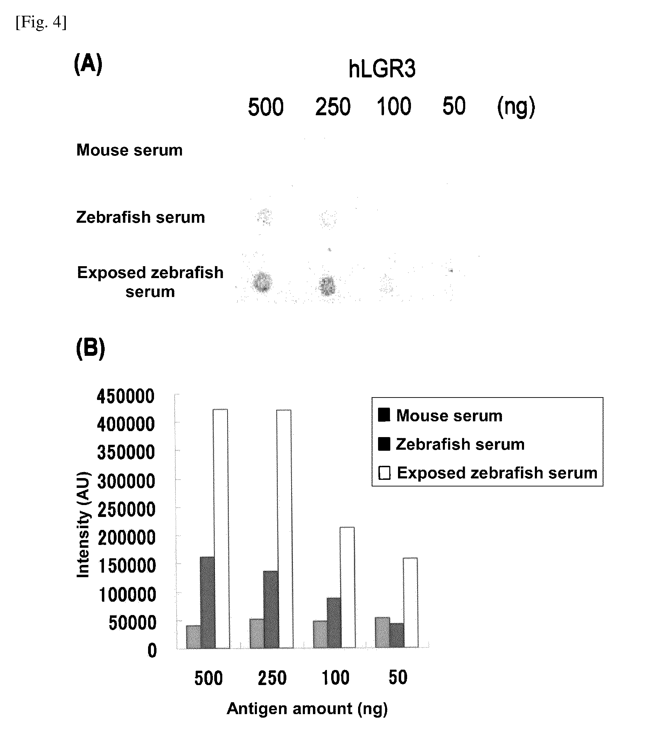

Using anti-zebrafish IgM antibody (provided by the laboratory of the present inventor) directly labeled with HRP (GE Healthcare), dot blotting was performed for sera collected from zebrafish following their exposure to antigen as described above in step 3. Non-immunized mouse and zebrafish sera were used for comparison.
As shown in Figure 4(A), significant spots (dots) were observed in a dot blot using human LGR3(LRR) (100 ng). As shown in Figure 4(B), quantification of fluorescence intensity revealed that the fluorescence was saturated when 250 ng or more of antigen protein, i.e., human LGR3(LRR), was used and that the fluorescence linearly correlates with the concentration of the protein when 100 ng or less protein was used.
Consequently, it was confirmed that anti-human LGR3 antibody is produced in the serum of zebrafish exposed to *E. coli* expressing human LGR3.

### INDUSTRIAL APPLICABILITY

According to the method of the present invention, anti-mammalian GPCR antibody, such as anti-human GPCR antibody, can be produced using fish. The production method is simple, and therefore, it easily produces anti-mammalian GPCR antibody. The anti-mammalian GPCR antibody obtained by this method may be a potential therapeutic antibody, diagnostic agent, and the like, whose target molecules are mammalian GPCRs, such as human GPCRs, to which the said antibody specifically binds. Moreover, such an antibody may be used in research of therapeutic antibodies, diagnostic agents, and the like.

## Claims

1. A method of producing anti-mammalian G protein-coupled receptor (GPCR) antibody, comprising a step of immunizing fish with full-length or truncated mammalian GPCR.

2. The method of producing anti-mammalian GPCR antibody according to claim 1, wherein the step of immunizing fish comprises a step of introducing an expression vector harboring a gene encoding the full-length or truncated mammalian GPCR into *Escherichia coli* or yeast and a step of exposing the fish to the *E. coli* or yeast.

3. The method of producing anti-mammalian GPCR antibody according to claim 1 or 2, wherein the mammal is human.

4. The method of producing anti-mammalian GPCR antibody according to any one of claims 1 to 3, wherein the mammalian GPCR is a human leucine-rich repeat-containing G protein-coupled receptor (human LGR).

5. The method of producing anti-mammalian GPCR antibody according to claim 4, wherein the human LGR is human LGR3 or LGR4.

6. The method of producing anti-mammalian GPCR antibody according to any one of claims 1 to 5, wherein the fish is either zebrafish, goldfish, Carassius, or carp.

7. Anti-mammalian GPCR antibody that can be obtained by the production method comprising a step of immunizing fish with full-length or truncated mammalian GPCR.

8. Anti-mammalian GPCR antibody according to claim 7, wherein the step of immunizing fish is a step of exposing the fish to *E. coli* or yeast in which an expression vector containing a gene encoding the full-length or truncated mammalian GPCR had been introduced.

9. Anti-mammalian GPCR antibody according to claim 7 or 8, wherein the mammalian GPCR is a human LGR.

10. Anti-mammalian GPCR antibody according to claim 9, wherein the mammalian GPCR is human LGR3 or human LGR4.

11. Anti-human GPCR antibody according to any one of claims 7 to 10, wherein the fish is either zebrafish, goldfish, Carassius, or carp.

12. A method of detecting anti-human GPCR antibody comprising use of fish IgM antibody directly labeled with horseradish peroxidase (HRP).
